# EUROPEAN PATENT SPECIFICATION

(11) **EP 3 281 662 B1**
(45) Date of publication and mention of the grant of the patent: **03.04.2019**
(21) Application number: 17185064.7
(22) Date of filing: 07.08.2017
(51) Int. Cl.: A61M 15/00, F03G 7/06

(54) **LINEAR ACTUATOR**
LINEARER AKTUATOR
ACTIONNEUR LINÉAIRE

(30) Priority: 08.08.2016 US 201662371988 P
(43) Date of publication of application: 14.02.2018
(73) Proprietor: Schneider Electric Controls UK Limited, Plymouth, Devon PL6 6QT (GB)
(72) Inventor: Allen, Owen Benjamin Chapman, Plymouth, PL2 1AP (GB); Clark, Lyndon James, Lifton, PL16 OLB (GB)
(74) Representative: Friese Goeden Patentanwälte PartGmbB

(56) References cited:
- FR-A5- 2 094 876
- US-A- 3 951 009
- US-A1- 2006 060 192
- US-B1- 9 297 448

## Description

### CROSS-REFERENCE TO RELATED APPLICATION

### TECHNICAL FIELD

The present disclosure generally relates to linear actuators that include both automatic and manual actuation drivers.

### BACKGROUND

Linear actuators include automatic drivers that use an external power source such as electricity, a pressurized fluid system (e.g., a hydraulic system, a pneumatic system, etc.), or the like to drive movement of an output part of the actuator along an axis. The output part of the actuator is mechanically linked to an input part of an actuatable device to drive corresponding movement of the input part and selectively actuate the device. For example, some linear actuators use electrically powered wax motors to drive movement of a piston assembly along an actuation axis through a range of motion that includes an actuating position and a non-actuating position. In certain embodiments, the piston assembly is operatively linked to a valve stem of a valve to drive movement of the valve stem that is operative to selectively open and close the valve. A manual driver may be included in a linear actuator in combination with the automatic driver. The manual driver allows a user to selectively actuate the actuatable device using the linear actuator even when the linear actuator is cut off from the external power source. For example, a user may access the manual driver to drive movement of a piston assembly when electrical power is cut off from a wax motor.

US 9 297 448 discloses a linear actuator according to the preamble of claim 1.

### SUMMARY

In one aspect, a linear actuator comprises a support. An output member is movably mounted on the support and selectively movable relative to the support through a range of motion including an actuating position and a non-actuating position. The output member is configured for being mechanically connected to a movable input member of an actuatable device to position the input member at an actuated position. A driver configured to selectively drive movement of the output member between the actuating position and the non-actuating position. An indicator is constrained for movement with the output member relative to the support. The indicator is positioned so that it is visible from a vantage external to the support when the output member is in one of the actuating position and the non-actuating position, and the indicator is positioned so that it is concealed by the support from said vantage when the output member is in the other of the actuating position and the non-actuating position.

In another aspect, a linear actuator for selectively actuating an actuatable device comprises a support. A piston is mounted on the support for movement generally along an actuation axis through a range of motion including an actuating position and a non-actuating position. The piston is movable in a first axial direction extending from the actuating position toward the non-actuating position and a second axial direction opposite the first axial direction. The piston includes at least one retainer element and is configured to be mechanically connected to a movable input member of the actuatable device to position the input member at an actuated position. An automatic driver is configured to selectively drive movement of the piston to the actuating position and the non-actuating position using power supplied by a power source. A manual driver is configured to selectively drive movement of the piston to the actuating position and the non-actuating position using a manual force. The manual driver comprises a manual input member rotatably mounted on the support for selective rotation about the axis with respect to the support. Aa linking member is threadably engaged with the manual input member for movement relative to the manual input member along the actuation axis when the manual input member is rotated relative to the linking member about the axis. The linking member includes at least one retainer formation configured for interlocking engagement with the at least one retainer element of the output assembly. The interlocking engagement between the retainer formation and the retainer element being operative to inhibit relative rotation between the piston and the linking member about the actuation axis, inhibit movement of the piston relative to the linking member along the axis in one of the first and second axial directions, and permit movement of the piston relative to the linking member along the axis in the other of the first and second axial directions.

Other aspects will be apparent hereinafter.

### BRIEF DESCRIPTION OF THE DRAWINGS

FIG. 1 is a perspective of a linear actuator according to an embodiment of the invention;
FIG. 2 is an exploded perspective of the linear actuator;
FIG. 3 is a longitudinal section of the linear actuator;
FIG. 4 is a perspective of an indicator member of a piston assembly of the linear actuator;
FIG. 5 is an end elevation of the indicator member;
FIG. 6 is a cross-section of the indicator member taken in the plane of line 6-6 of FIG. 5;
FIG. 7 is a perspective of a manual input cap of the linear actuator;
FIG. 8 is a longitudinal section of the manual input cap;
FIG. 9 is a perspective of a linking collar of the linear actuator;
FIG. 10 is an end elevation of the linking collar;
FIG. 11 is a cross-section taken in the plane of line 11-11 of FIG. 10;
FIG. 12 is cross-section of a subassembly of the linear actuator including the indicator member and the linking collar operatively received therein extending in a plane oriented orthogonal to an actuation axis of the linear actuator at a location that intersects the indicator member and the linking collar;
FIG. 13 is a fragmentary longitudinal cross-section of the sub-assembly illustrated in FIG. 12;
FIG. 14A is a longitudinal cross-section of another sub-assembly of the linear actuator including the manual input cap, the linking collar, the indicator member, and a wax motor of the linear actuator, illustrating the configuration of the subassembly when the linear actuator is in a non-actuating configuration;
FIG. 14B is a cross-section similar to FIG. 14A, illustrating the configuration of the sub-assembly of FIG. 14A when the linear actuator is manually configured to actuate an actuatable device;
FIG. 14C is a cross-section similar to FIG. 14A, illustrating the configuration of the sub-assembly of FIG. 14A when the linear actuator is automatically configured to actuate an actuatable device;
FIG. 15A is another perspective of the linear actuator, illustrating the transparency of the manual input cap and illustrating a non-actuating configuration;
FIG. 15B is a perspective similar to FIG. 15A, illustrating an actuated configuration in which the indicator member is visible through the manual input cap; and
FIG. 16 is an exploded perspective of a subassembly of the linear actuator including a housing member and an output member.

Corresponding reference characters indicate corresponding parts throughout the drawings.

### DETAILED DESCRIPTION

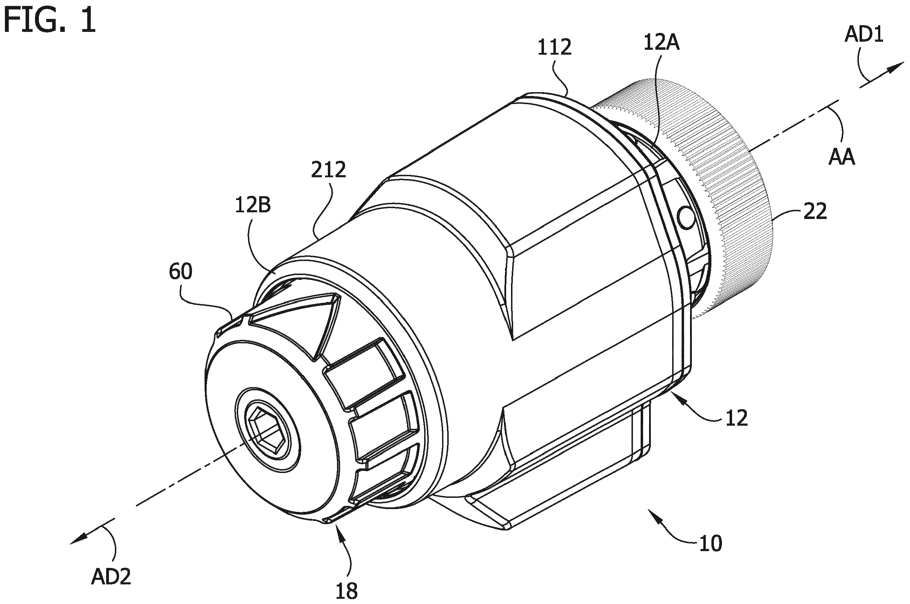

Referring to FIGS. 1-3, one embodiment of a linear actuator is generally indicated at reference number 10. As is known in the art, the illustrated linear actuator 10 is configured to selectively drive movement of a movable input component of an actuatable device to selectively actuate the device. For example, the linear actuator 10 may drive movement of a valve stem (broadly, an input component or input member; not shown) of a valve (broadly, an actuatable device; not shown) to selectively open and close the valve. As explained below, the illustrated linear actuator 10 can be operated automatically using an external power source or manually by a user. As will be apparent, the manual actuation mechanism of the illustrated linear actuator 10 can be used to selectively actuate an acuatable device using only a relatively small amount of manually applied force. In addition, when either the manual drive mechanism or the automatic drive mechanism is used, the linear actuator 10 provides a clear visual indication of the location of the output part of the linear actuator, which provides an indication of the actuation state of the linked actuatable device.

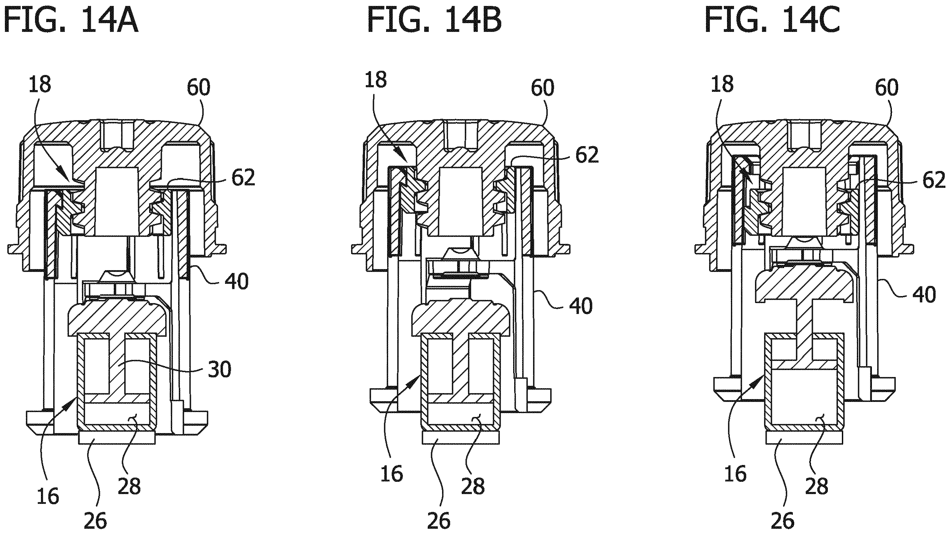

The linear actuator 10 includes a housing or casing (broadly, a support), generally indicated at 12. The housing 12 supports a piston assembly, generally indicated at 14, for movement with respect to the housing along an actuation axis AA. As explained below, the piston assembly 14 forms the output part of the illustrated linear actuator 10. Movement of the piston assembly 14 relative to the housing 12 drives movement of the input part of the actuatable device operative to selectively actuate the device. An automatic driver, generally indicated at 16, is received in the housing 12 and is operatively connected to the piston assembly 14 to drive movement of the piston assembly relative to the housing along the actuation axis AA. As explained below, the automatic driver 16 is configured to move the piston assembly 14 relative to the housing 12 through a linear or axial range of motion that includes an actuating position (FIG. 14C) and a non-actuating position (FIG. 14A). From the actuating position, the piston assembly 14 moves along the actuation axis AA in a first, non-actuating axial direction AD1 to the non-actuating position; and from the non-actuating position, the piston assembly moves along the actuation axis in a second, actuating axial direction AD2 toward the actuating position. It is understood that if the orientation of the linear actuator 10 with respect to the actuatable device were reversed, the first axial direction AD1 would be an actuating direction and the second axial direction AD2 would be a non-actuating direction. A manual driver, generally indicated at 18, is secured to the housing 12 and operatively connected to the piston assembly 14 to drive movement of the piston assembly relative to the housing generally along the actuation axis AA between the non-actuating position and a manually positioned actuating position shown in FIG. 14B. As will be explained in further detail below, the manual driver includes a manual input component that is movably mounted on the housing 12 to receive a manually applied force that drives movement of the piston assembly 14 between the actuating and non-actuating positions.

Referring still to FIGS. 1-3, the housing 12 extends along the actuation axis AA from a first axial end portion 12A to an open second axial end portion 12B spaced apart from the first axial end portion in the second axial direction AD2. For reasons explained below, the housing 12 is suitably made from an opaque material. In the illustrated embodiment, the housing 12 includes a first external housing member 112 and a second external housing member 212 that are configured to be secured to one another to define an actuator chamber 20. An actuator mount 22 is secured to the first housing member 112 adjacent the first end portion 12A of the housing 12. The mount 22 is configured to support the linear actuator 10 on an underlying support structure in use. Suitably, a gasket is placed at the interface between the first and second external housing members 112, 212 to seal the actuator chamber 20 from environmental contaminants.

In the illustrated embodiment, the first and second external housing members 112, 212 have mating interlocking features that secure the housing members together. In other embodiments, it is understood that the housing members are secured together in other ways (e.g., using fasteners). In addition to the first and second external housing members, 112, 212 the illustrated housing 12 includes an internal housing member 312 that is secured between the first and second external housing members 112, 212 in the actuator chamber 20. Like the second external housing member 212, the internal housing member 312 includes features that interlock with mating features of the first external housing member 112 to secure the internal housing member in place inside the chamber 20. The internal housing member can be secured to the external housing members in other ways in other embodiments.

Each of the second external housing member 212 and the internal housing member 312 has an open end adjacent the second end portion 12B of the housing 12 that is spaced apart from the first external housing member 112 along the actuation axis AA. As explained below, the open ends of the housing members receive a portion of the manual driver that protrudes outward from the actuator chamber 20 in the second axial direction AD2. The exposed portion of the manual driver 18 allows a user to provide a manual input force to the manual driver for driving movement of the piston assembly 14 in the actuating and non-actuating directions AD1, AD2.

Although the illustrated embodiment uses a housing 12 comprising two external housing members 112, 212 and one internal housing member 312, it will be understood that other linear actuators can have supports having other constructions without departing from the scope of the invention. For example, it is contemplated that in one or more embodiments, the linear actuator is non-enclosed and the support does not define an enclosed chamber.

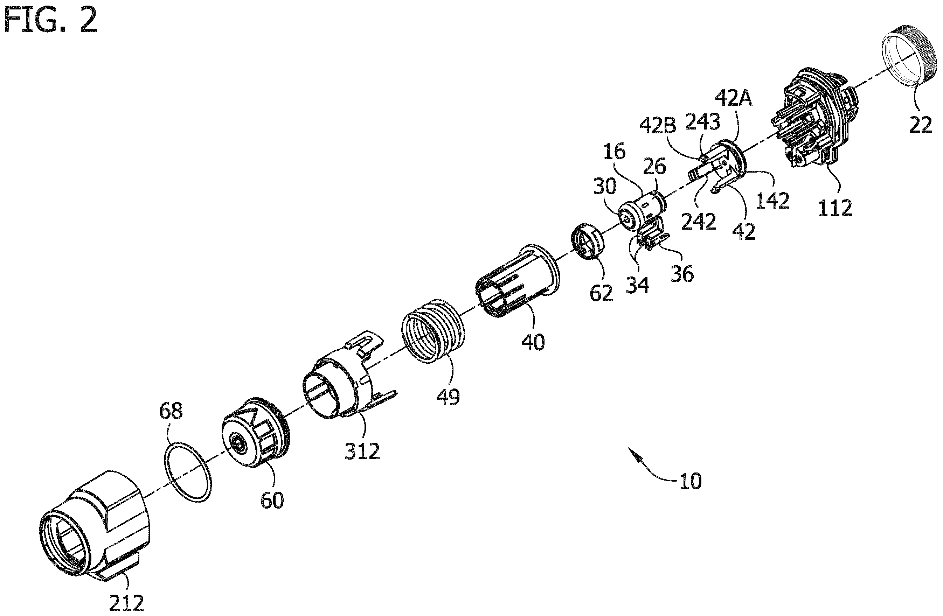

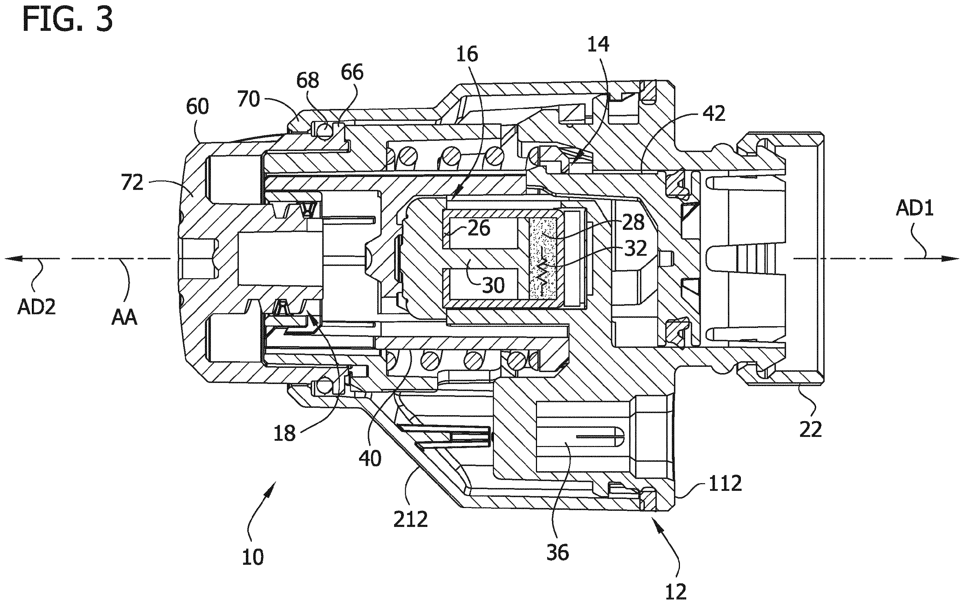

Referring to FIGS 2 and 3, in the illustrated embodiment, the automatic driver 16 is a thermal driver comprising a wax motor. As is known in the art, the wax motor 16 includes an enclosure 26 that contains a wax 28 or another thermally expandable material. A plunger 30 is slidably secured to the enclosure 26 and operatively coupled to the wax 28 such that, when the volume of wax in the enclosure changes due to a thermal input, the plunger moves along the actuation axis AA relative to the enclosure in one of the first actuation direction AD1 and the second actuation direction AD2. A heater 32 (e.g., a resistive heater) is operatively coupled to the wax 28 to heat the wax when electrical power is selectively applied to the heater. The heater 32 includes rigid lead members 34 that are electrically coupled to heating elements (e.g., electrical resistors, not shown) of the heater and to electrical contacts 36 configured for mating connection with a plug receptacle (not shown). The rigid lead connectors are rigidly connected to the housing 12 and to the enclosure 26 and thereby fix the enclosure in place with respect to the housing. Thus, when the plunger 30 moves relative to the enclosure 26, it likewise moves relative to the housing 12. As explained below, the piston assembly 14 is constrained to move with plunger 30 such that movement of the plunger relative to the enclosure drives movement of the piston assembly relative to the housing 12.

Although the illustrated linear actuator 10 uses a wax motor 16 to automatically drive movement of the piston assembly 14 through its range of motion, other embodiments can include other types of automatic linear drivers. For example, other types of linear thermal drivers, linear electrical motor drivers, linear pneumatic drivers, and linear hydraulic drivers are used in respective embodiments. It is understood that an "automatic" driver differs from a "manual" driver because it uses power (e.g., electricity, fluid pressure, etc.) other than forces imparted by a human user (with or without an applied mechanical advantage) to drive movement of a piston assembly of the actuator.

In the illustrated embodiment, the piston assembly 14 comprises an indicator member 40 and an output member 42 that are constrained to move together along the actuation axis AA with respect to the housing 12 in response to a driving force imparted by either the wax motor 16 or the manual driver 18. As explained below, the indicator member 40 is configured to receive the drive forces imparted by the automatic and manual drivers 16, 18 and thereby move the piston assembly 14 with respect to the housing 12. Suitably, the output member 42 is operatively linked to the input member of an actuatable device (e.g., a valve stem of a valve) such that movement of the output member drives corresponding movement of the input member.

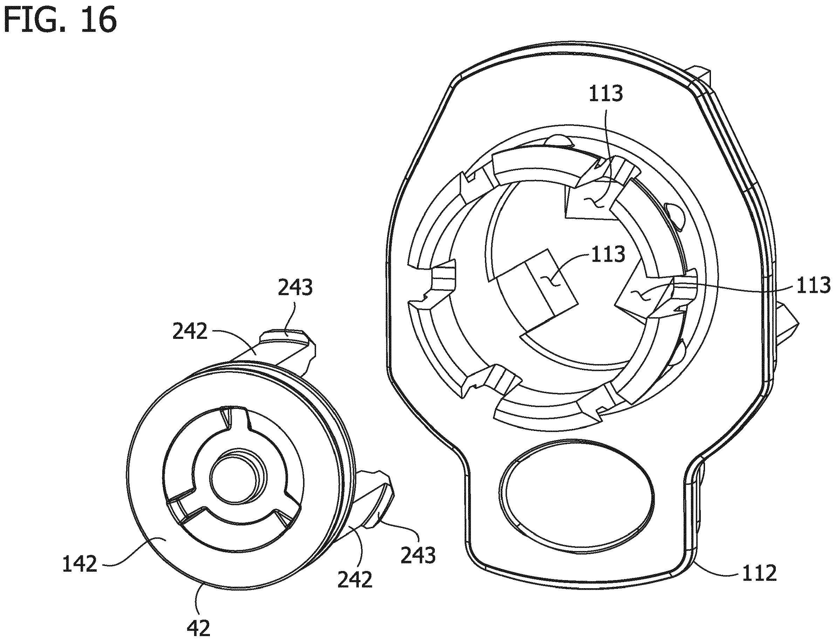

As shown in FIG. 2, the output member 42 has a first end portion 42A and an opposite second end portion 42B spaced apart from the first end portion along the actuation axis AA in the second axial direction AD2. In the illustrated embodiment, the output member 42 includes a base portion 142 that forms the first end portion 42A and three prongs 242 that extend generally along the actuation axis AA from the base portion in the second axial direction AD2 to form the second end portion 42B of the output member. The prongs 242 are circumferentially spaced apart from one another about the actuation axis AA and define gaps therebetween. The rigid lead members 34 of the wax motor 16 extend through the one or more of the gaps between the prongs 242 without contacting the piston assembly 14 so that the piston assembly can move freely along the actuation axis AA with respect to the rigid lead members. As shown in FIG. 16, the prongs 242 are shaped and arranged for being slidably received in corresponding holes 113 that extend axially through the first housing member 112 at circumferentially spaced apart locations about the actuation axis AA. When the prongs 242 are received in the holes 113 in the first housing member 112, they secure the output member 42 to the first housing member against rotation with respect to the first housing member about the actuation axis AA. In addition, by extending through the holes 113 in the first housing member 112, the prongs 242 allow the base portion 142 of the output member 42 to be positioned on the exterior of the first housing member, which exposes the output member for operative connection with an input member of an actuatable device. Suitably the prongs 242 have sufficient length along the actuation axis AA to allow the prongs to slide through the holes 113 in the first housing member 112 through the entire range of motion of the piston assembly 14 without the base portion 142 interfering with the housing 12.

The free ends of the prongs 242 at the second end portion 42B of the output member 42 define catches 243 configured for interlocking engagement with the indicator member 40. When the catches 243 are lockingly engaged with the indicator member 40, they fix the output member 42 in place with respect to the indicator member. That is, the catches 243 secure the output member 42 to the indicator member 40 so that the components of the piston assembly 14 are constrained to move as a single unit. Since the prongs 242 secure the output member 42 against rotation with respect to the housing 12 as explained above, the locking engagement between the catches 243 and the indicator member 40 prevents rotation of the indicator member with respect to the housing when the prongs are received in the holes 113 of the first housing member 112.

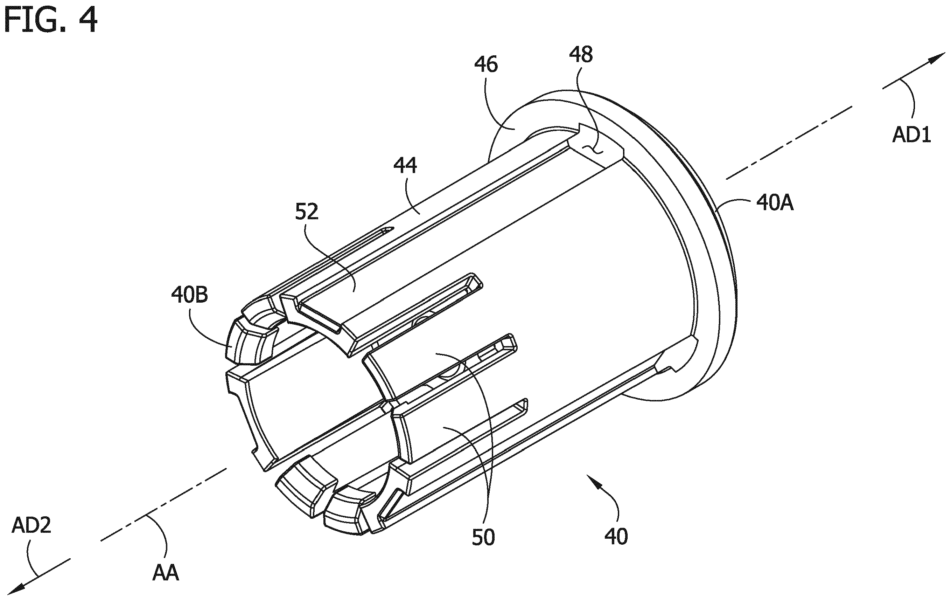

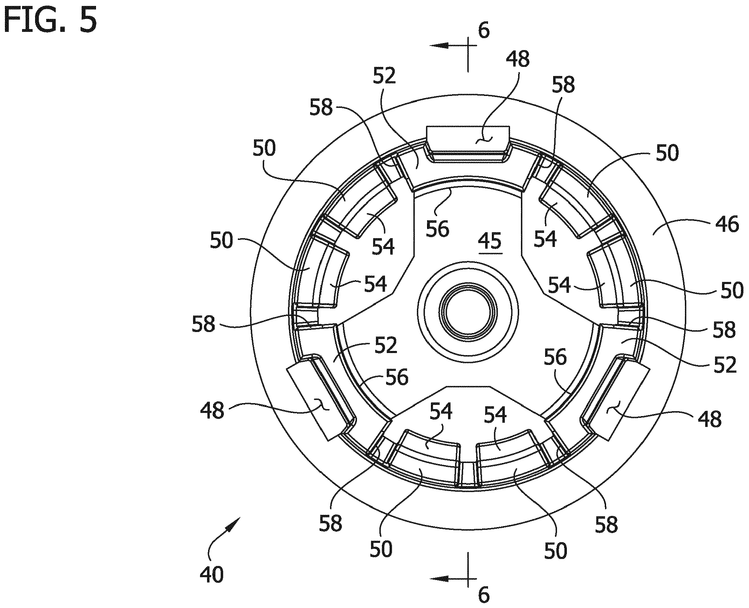

Referring to FIGS. 4-6, the indicator member 40 has a first end portion 40A and an opposite second end portion 40B that is spaced apart from the first end portion along the actuation axis AA. An annular wall 44 extending circumferentially around the actuation axis AA extends from adjacent the first end portion 40A toward the second end potion 40B. An intermediate bearing wall 45 extends transverse to the annular wall 44 at an intermediate location between the first and second end portions 40A, 40B of the indicator member 40. The bearing wall 45 is configured to engage the outer end of the plunger 30 of the wax motor 16. When the plunger 30 moves in the second actuating direction AD2 with respect to the wax motor enclosure 26, the plunger bears against the intermediate bearing wall 45 and thereby urges the indicator member 40 to move with the plunger with respect to the housing 12.

The first end portion 40A of the indicator member 40 defines a flange 46 that extends radially outwardly from the annular wall 44 and defines axial shoulder facing in the second axial direction AD2. Three slots 48 are formed in the first end portion 40A of the indicator member 40 at circumferentially spaced apart positions about the actuation axis AA that correspond with the positions of the prongs 242 of the output member 42. The slots 48 are shaped and arranged to receive the catches 243 of the prongs 242 so that the catches lockingly engage the first end portion 40A of the indicator member 40 to constrain the output member 42 and the indicator to move together as a single piston assembly unit with respect to the housing 12.

Referring to FIG. 3, the flange 46 of the indicator member 40 functions as a spring retainer for engaging a spring 49, which biases the piston assembly 14 toward the non-actuating position (FIG. 14B). Although the illustrated linear actuator 10 is biased toward the non-actuating position, it will be understood that other embodiments are biased toward the actuating position instead of the non-actuating position. One end of the spring 49 engages the shoulder defined by the flange 46 of the indicator member 40 and an opposite end of the spring engages an opposing axial surface of the internal housing member 312. The spring 49 imparts a force between the internal housing member 312 and the indicator member 40 that urges the piston assembly 14 in the first, non-actuating direction AD1, toward the non-actuating position. Thus, in order to position the piston assembly 14 in the actuating position, a force imparted on the piston assembly in the second, actuating direction AD2 must overcome the biasing force of the spring 49.

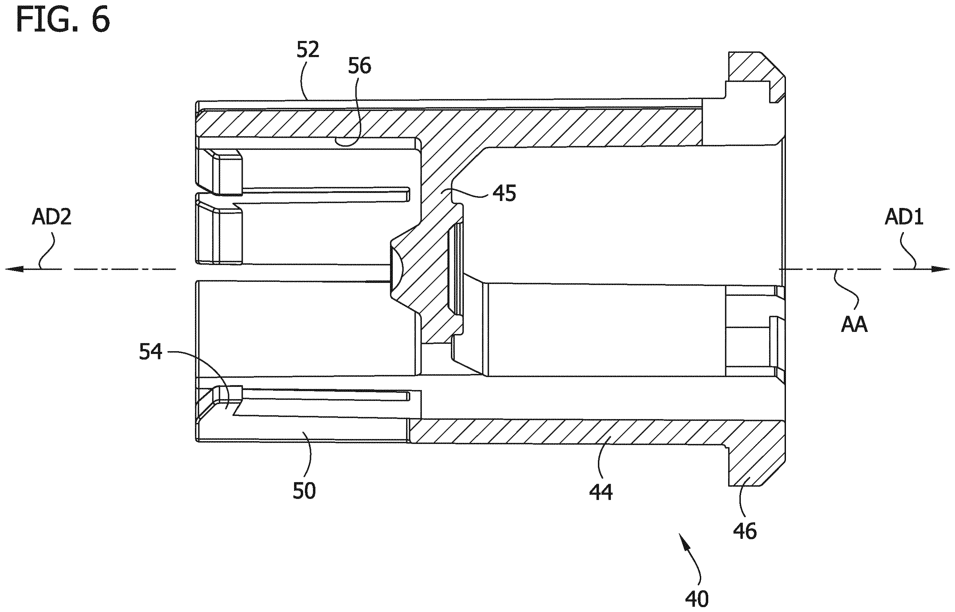

Referring again to FIGS. 4-6, the second end portion 40B of the indicator member 40 defines a plurality of axially extending fingers 50, 52 that extend along the actuation axis AA in the second, actuating direction AD2 from the annular wall 44 and are circumferentially spaced apart from one another about the actuation axis. As explained below, the fingers 50, 52 are suitably made from an opaque material so that the fingers can be used to provide a visual indication of the position of the piston assembly 14 in the housing 12. In the illustrated embodiment, the indicator member 40 includes fingers 50 that function as axial retainers as explained below and fingers 52 that function as circumferential retainers as explained below. As shown in FIG. 6, the end portion of each of the axial retainer fingers 50 includes a radially inwardly extending catch 54 that defines an inner radial end of the finger. As explained below, each catch 54 includes an axial end surface facing in the first axial direction AD1 that is configured to engage an opposing axial surface of the manual driver 18 to provide a stop for inhibiting the indicator member 40 from moving relative to the manual driver in the first, non-actuating direction when the surfaces are engaged. In the illustrated embodiment, the surface defining the axial stop is skewed with respect to the actuation axis AA at an angle that extends in the first axial direction AD1 as it extends inward toward the actuation axis. It is understood that, in other embodiments the surface defining the axial stop has other orientations.

Each of the circumferential retainer fingers 52 has an inner radial surface 56 that extends substantially parallel to the actuation axis along the entire axial extent of the finger. That is, unlike the axial retainer fingers 50, the circumferential retainer fingers 52 do not define radially extending catches or other inwardly projecting radial protrusions. Suitably, the inner radial surfaces 56 of the circumferential retainer fingers 52 have about the same radial positions as the inner radial ends of the catches 54 of the axial retainer fingers 50. Each of the circumferential retainer fingers 52 has first and second circumferential side surfaces 59 that are circumferentially spaced apart from one another and face in generally opposite circumferential directions. As explained below, each side surface 59 is configured to function as a circumferential stop that engages an opposing surface of the manual driver 18 to inhibit the indicator member 40 from moving relative to the manual driver in a respective circumferential direction about the actuation axis AA.

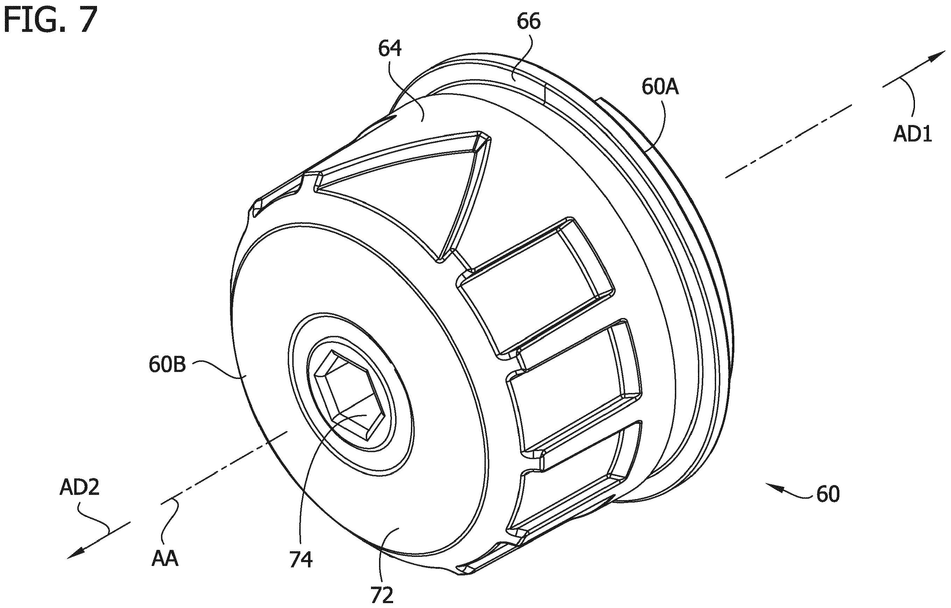

Referring again to FIGS. 2-3, the manual driver 18 includes a manual input cap 60 (broadly, a manual input member or knob) and a linking collar 62 that is threadably engaged with the manual input cap as explained below. As shown in FIGS. 7-8, the manual input cap 60 has an open first end portion 60A, an opposite closed second end portion 60B that is spaced apart from the first end portion along the actuation axis AA in the second axial direction AD2, and an annular wall 64 that extends along the actuation axis from adjacent the first end portion toward the second end portion. The first end portion 60A of the manual input cap 60 defines a radially outwardly extending flange 66. As shown in FIG. 3, an axial surface of the flange 66 that faces in the second axial direction AD2 forms a shoulder with the annular wall 64 of the manual input cap that defines seat for engaging a seal 68. In the assembled linear actuator 10, the annular wall 64 of the manual input member 60 extends through the open end of the second housing member 212 and the seal 68 is compressed between the flange 66 of the manual input member and an opposing radially inwardly extending flange 70 of the second housing member. An axial surface of the flange 66 of the manual input member 60 that faces in the first axial direction AD1 also engages an opposing axial surface of the internal housing member 312. Thus, the flange 66 of the manual input member 60 is captured between the internal housing member 312 and the second external housing member 212 and seal 68. The internal housing member 312, the second external housing member 212, and the seal 68 act together to hold the manual input cap 60 in the proper axial position with respect to the housing 12. Thus, the housing 12 mounts the manual input cap 60 to restrain relative axial movement between the manual input cap and the housing. But the housing 12 is not configured to restrict rotation of the manual input member 60 about the actuation axis AA with respect to the housing. Thus, the manual input cap 60 is rotatably mounted on the housing 12 of the linear actuator 10 for rotation about the actuation axis AA with respect to the housing.

The second end portion 60B of the manual input cap 60 forms a radially extending end wall 72 that intersects the actuation axis AA. A cavity 74 shaped and arranged for mating with a working end portion of a manual driver tool is formed in the end wall 72. In the illustrated embodiment, the cavity 74 is a hexagonal cavity configured for receiving a hexagonal bit. In other embodiments, other tool-receiving cavities (e.g., Phillips depressions, flathead slots, etc.) are used in place of the hexagonal cavity. The cavity 74 allows the manual input cap 60 to be rotated about the actuation axis AA with respect to the housing 12 using the manual driver tool. Alternatively, a user may grip the annular wall 64 of the cap 60 with a hand to rotate the cap about the actuation axis AA.

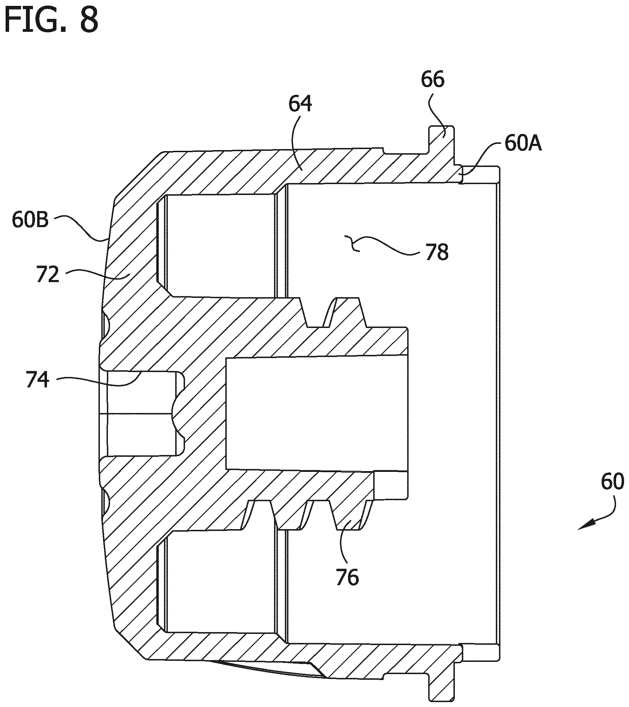

Referring to FIG. 8, an externally threaded post 76 extends in the first axial direction AD1 from the end wall 72 of the manual input cap 60. Suitably, the post 76 is generally centered on the actuation axis AA and is radially spaced apart from the annular wall 64 to define an annular gap 78 for receiving the linking collar 62, the second end portion 40A of the indicator member 40, and an end portion of the internal housing member 312 therein as shown in FIG. 3. In the illustrated embodiment, the post 76 is formed integrally with the rest the cap 60 from a single piece of material. In other embodiments, the post is separately attached to the end wall of the cap.

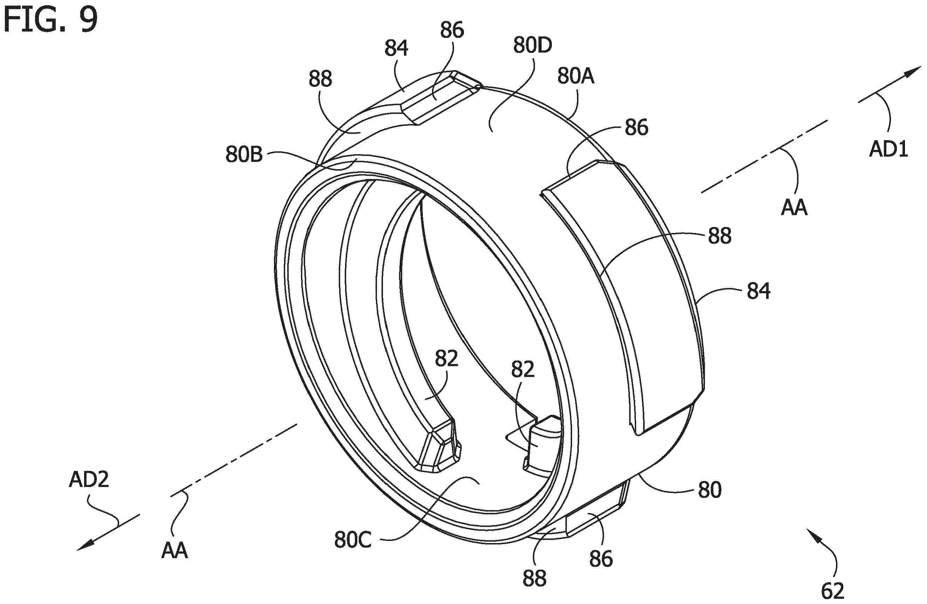

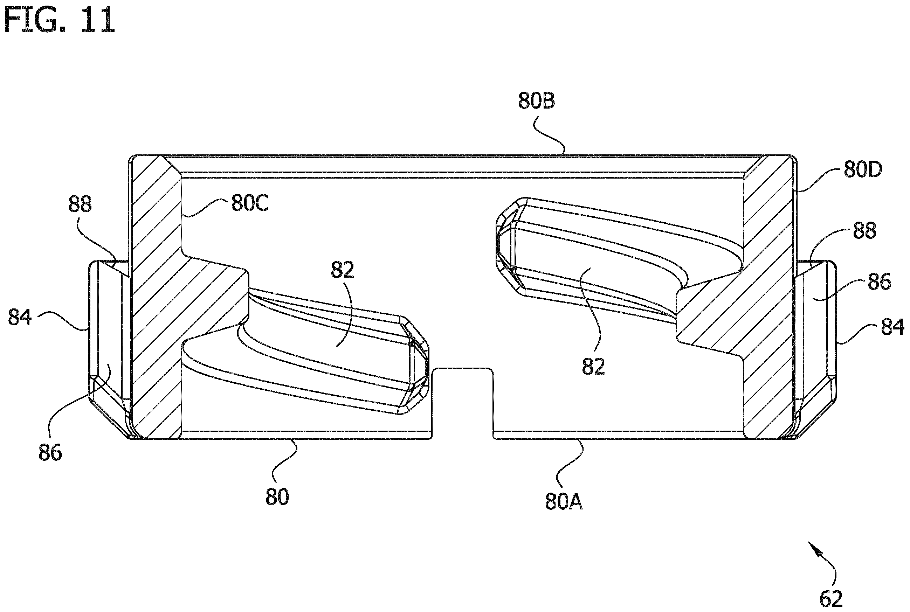

Referring to FIGS. 9-11, the linking collar 62 includes an annular body 80 having a first end portion 80A and an opposite second end portion 80 spaced apart from the first end portion along the actuation axis AA. The annular body 80 also has a radially inward facing surface 80C and an opposite radially outward facing surface 80D. Two circumferentially spaced apart semi-helical threads 82 extend radially inward from the inward facing surface 80C. The threads 82 are shaped and arranged to threadably engage the threaded post 76 such that relative rotation between the linking collar 62 and the manual input cap 60 about the actuation axis AA causes relative movement between the manual input cap and the linking collar along the actuation axis.

The outward facing surface 80D of the annular body 80 is shaped and arranged for being received in an opening in the second end portion 40B of the indicator member 40, radially inward of the fingers 50, 52. A plurality of circumferentially spaced apart retainer formations 84 project radially outward from the outward facing surface 80D of the annular body 80 at circumferentially spaced apart positions. In the illustrated embodiment, the linking collar 62 includes three retainer formations 84 of substantially equal dimensions, but one of the retainer formations includes a small circumferential gap at about a circumferential midpoint thereof.

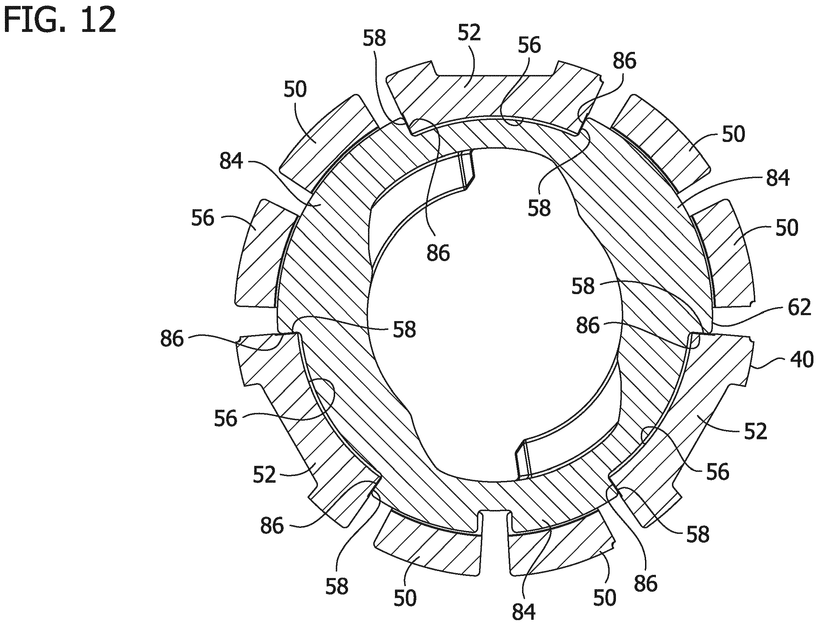

The retainer formations 84 are sized and arranged for being received in the gaps between the inner radial portions of the circumferential retainer fingers 52 of the indicator member 40 (i.e., in circumferential alignment with the axial retainer fingers 50) as shown in FIG. 12. Each retainer formation 84 has first and second circumferential side surfaces 86 that opposingly engage the circumferential side surfaces 58 of a respective pair of adjacent circumferential retainer fingers 52 as shown in FIG. 12. In this way, the retainer formations 84 are keyed to the circumferential retainer fingers 52, and the engagement between the side surfaces 86 of the retainer formations and the side surfaces 58 of the circumferential retainer fingers prevents relative rotation between the linking collar 62 and the indicator member 40 when the linking collar is keyed to the indicator member.

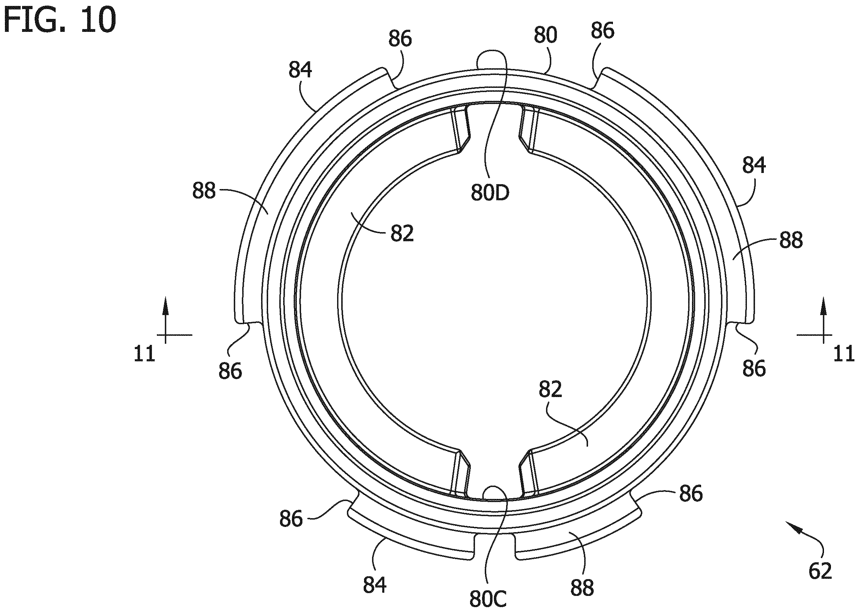

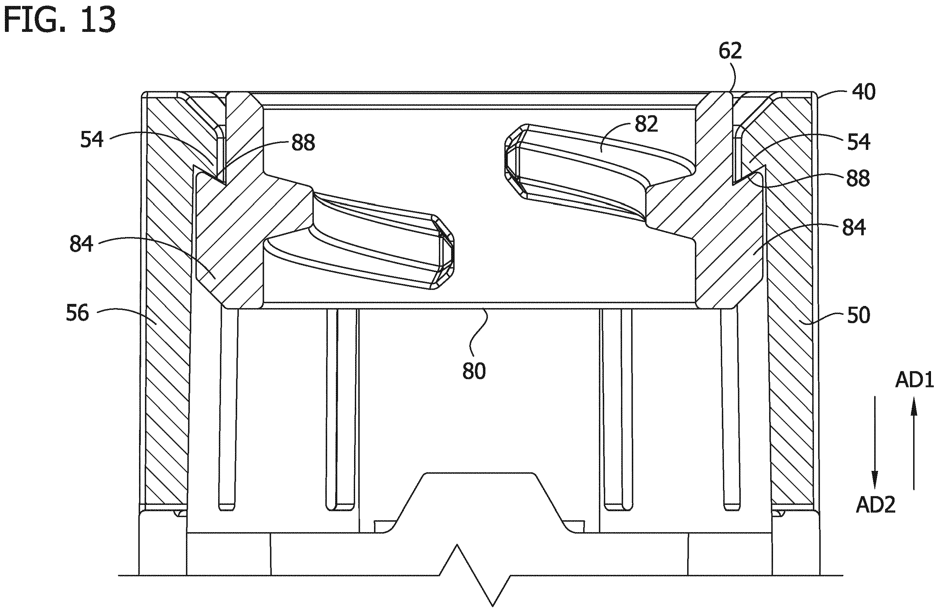

Referring to FIGS. 10, 11, and 13, each retainer formation 84 also has an axial end surface 88 that faces in the second axial direction AD2 and that is shaped and arranged for engagement with the catches 54 of a respective pair of axial retainer fingers 50. In the illustrated embodiment, the axial end surface 88 is skewed with respect to the actuation axis AA at an angle that extends in the first axial direction AD1 as it extends inward toward the actuation axis. Thus the axial end surfaces 88 of the retainer formations 84 are shaped and arranged to conform to the opposing axial end surfaces of the catches 54 of the axial retainer fingers 50. As shown in FIG. 13, when axial end surface 88 engages the opposing axial surface of the catch 54, the engagement prevents the indicator member 40 from moving in the first axial direction AD 1 with respect to the linking collar 62 and prevents the linking collar from moving in the second axial direction AD2 with respect to the indicator member 40. However, the indicator member 40 is free to move in the second axial direction AD2 with respect to the linking collar 62. As explained below, this allows the wax motor 16 to drive movement of the piston assembly 14 from the non-actuating position to the actuating position without interference by the manual driver 18.

Referring to FIGS. 14A-14C, in use either the manual driver 18 or the automatic driver 16 may be used to move the piston assembly 14 between the non-actuating position and the actuating position. For clarity, FIGS. 14A-14C illustrate only the indicator member 40 of the piston assembly 14. It is understood that, since the all of the components of the piston assembly 14 are constrained to move together with the indicator, and since (as explained below) both the manual driver 18 and the automatic driver 16 bear directly on the indicator member 40 to drive movement of the piston assembly, the position of the indicator member determines the position of the piston assembly. Thus the positions of the indicator member 40 in FIGS. 14A-14C are representative of respective positions of the piston assembly 14.

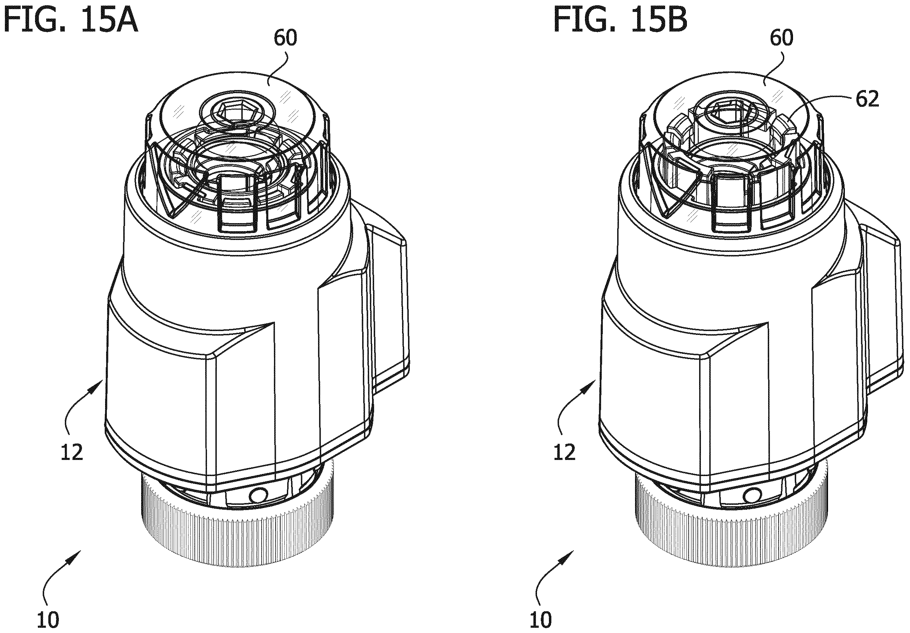

Figures 3 and 14A illustrate the piston assembly 14 in the non-actuating position. As shown in FIG. 3, in the non-actuating position, the second end portion 40B of the indicator member 40 is axially aligned with the second end portion of the linear actuator housing 12. Since the housing 12 is opaque, it substantially conceals the radially outward facing surface of the indicator member 40 from view through the transparent input member cap 60 when the piston assembly 14 is in the non-actuating position, as shown in FIG. 15A.

Referring to FIGS. 14A-14B, to manually move the piston assembly 14 from the non-actuating position (FIG. 14A) to a manually derived actuating position (FIG. 14B), the manual input cap 60 is rotated in a first direction about the actuating axis (e.g., an actuating direction), either by hand or using a manual driver tool as explained above. The threaded post 76 of the manual input cap 60 threadably bears against the helical internal threads 82 of the linking collar 62. Since the manual input cap 60 is axially restrained by the housing 12, the linking collar 62 is rotationally restrained by the indicator member 40, and the indicator member is rotationally restrained by the output member 42 and housing 12 as explained above, rotation of the manual input cap 60 with respect to the housing 12 drives the linking collar 62 in the second axial direction AD2 with respect to the manual input cap 60 and the housing. The axial end surfaces 88 of the retainer formations 84 of the linking collar 62 bear against the catches 54 of the axial retainer fingers 50 of the indicator member 40 to drive corresponding movement of the indicator member 40 and thus the piston assembly 14 in the second axial direction AD2 with respect to the housing 12. In the actuating position, the output member 42 of the piston assembly 14 positions the input member 60 of the actuatable device in an actuated position whereby the device is actuated (e.g., a valve is opened, etc.).

As shown in FIGS. 14B and 15B, when the piston assembly 14 is positioned in the actuating position, the second end portion 40B of the indicator member 40 protrudes beyond the open second end portion 12A of the housing 12 in the second axial direction AD2. As a result, the second end portion 40B of the indicator member 40 is axially aligned with the transparent annular wall 64 of the manual input cap 60. The radially outward facing surface of the second end portion 40B of the indicator member 40 is thus visible through the manual input cap 60 and provides a visual indication that the linear actuator 10 is in an actuating configuration.

Although in the illustrated embodiment the indicator member 40 is visible only when the piston assembly 14 is in the actuating position, it will be understood that the orientation of the linear actuator 10 could be reversed so that the indicator member 40 is visible through the indicator only when the piston assembly is in the non-actuating position.

After the manual driver 18 has been used to manually position the piston assembly 14 in the actuating position, the manual driver must again be used to reposition the piston assembly in the non-actuating position because the manual input cap 60 holds the linking collar 62 in a position in which the retainer formations 84 otherwise interfere with movement of the indicator member 40 in the first axial direction AD1 with respect to the housing 12. To manually return the piston assembly 14 to the non-actuating position, the user rotates the manual input member 60 in the opposite direction about the actuation axis AA. The threaded post 76 of the manual input member 60 threadably engages the internal threads 82 of the linking collar 62, and the screw action drives the collar in the first axial direction AD1 with respect to the input cap 60. The spring 49 bears against the spring retainer flange 46 of the indicator member 40 to urge the indicator member 40 to move with the linking collar 62 in the first axial direction AD1. The cap 60 is rotated until the radially outward facing surface of the second end portion 40B of the indicator member 40 is no longer visible through the cap, at which point the piston assembly 14 is in the non-actuating position. In the non-actuating position, the output member 42 of the piston assembly 14 positions the input member 60 of the actuatable device in a non-actuated position in which the device is not actuated (e.g., the valve is closed).

Referring to FIGS. 14A and 14C, the manual driver 18 is configured so that it does not interfere with the operation of the automatic driver 16 as it moves the piston assembly 14 between the non-actuating and actuating positions. When the automatic driver 16 is used to move the piston assembly 14 from the non-actuating position to the actuating position, power is supplied to the heater 32 to heat the wax 28. The heated wax 28 expands and drives the plunger 30 to move relative to the enclosure 26 in the second axial direction AD2. The plunger 30 bears against the intermediate wall 45 of the indicator member 40 to drive corresponding movement of the piston assembly 14 with respect to the housing 12. The retainer formations 84 of the linking collar 62 do not interfere with or opposingly engage any portion of the indicator member 40 as it moves relative to the housing 12 from the non-actuating position in the second axial direction AD2. Likewise, no other portion of the linking collar 62 or manual input cap 60 interferes with or opposingly engages the indicator member 40 as it moves relative to the housing 12 in the second axial direction AD2 to the actuating position. Instead, the retainer formations 84 are slidably received between the circumferential retainer fingers 52 so that the indicator member 40 slides axially along the linking collar 62 in the second axial direction AD2 in response to the actuating force imparted by the wax motor 16. As explained above with respect to the manually driven actuating position shown in FIG. 14B, when the automatic driver 16 moves the piston assembly 14 to the automatically driven actuating position shown in FIG. 14C, the radially outward facing surface of the second end portion 40B of the indicator member 40 is visible through the annular wall 64 of the transparent manual input member cap 60 as shown in FIG. 15B. From a vantage outside the linear actuator 10 as shown in FIG. 15B, the visual indication that the linear actuator is in the actuating configuration appears the same, whether the manual driver 18 or the automatic driver 16 was used to position the piston assembly 14 in the actuating position.

To return the piston assembly 14 to the non-actuating position from the actuating position using the automatic driver 16, electrical power to the heater 32 is cut off. As a result, the wax 28 cools and contracts in volume. At the urging of the spring 49 against the indicator member 40, the indicator member is forced in the first axial direction AD1 and bears against the plunger 30 to urge the plunger in the first axial direction with respect to the wax motor enclosure 26. The piston assembly 14 moves in the first axial direction AD1 relative to the housing 12 at the same rate as the plunger until it reaches the non-actuating position.

When introducing elements of the present invention or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects of the invention are achieved and other advantageous results attained. As various changes could be made in the above constructions, products, and methods without departing from the scope of the invention as defined by the appended claims.

### PARTS LIST

AA - actuation axis
AD1 - first actuation direction
AD2 -second actuation direction
10 - linear actuator
12 - housing (broadly, a support)
14 - piston assembly
16 - automatic driver (wax motor)
18 - manual driver
12A - first axial end portion of housing
12B - second axial end portion of housing
112 - first external housing member
113 - prong-receiving holes in first external housing member
212 - second external housing member
20 - actuator enclosure
312 - internal housing member
22 - actuator mount
26 - wax motor enclosure
28 - wax
30 - plunger
32 - heater
34 - rigid lead members
36 - electrical contacts
42- output member
42A - first end portion of output member
42B - second end portion of output member
142 - base portion of output member
242 - prongs of output member
243 - catches of prongs
40 - indicator member
40A - first end portion of indicator member
40B - second end portion of indicator member
44 - annular wall of indicator member
45 - intermediate bearing wall
46 - flange of indicator member
48 - slots of indicator member
49 spring
50 - axial retainer fingers
52 - circumferential retainer fingers
54 - catch of axial retainer finger
56 - inner radial surface of circumferential retainer fingers
58 - circumferentially spaced apart side surfaces of circumferential retainer fingers
60 - manual input cap
62 - linking collar
60A - first end portion of manual input cap
60B - second end portion of manual input cap
64 - annular wall of manual input cap
66 - flange of manual input cap
68 - seal
70 - radially inwardly extending flange of the first housing member
72 - end wall of manual input cap
74 - cavity in end wall of manual input cap
76 - externally threaded post
78 - annular gap in manual input cap
80 - annular body of linking collar
80A - first end of annular body of linking collar
80B - second end of annular body of linking collar
80C - radially inward facing surface of annular body of linking collar
80D - radially outward facing surface of annular body of linking collar
82 - semi helical threads
84 - retainer formation
86 - circumferential side surface of retainer formation
88 - axial end surface of retainer formation

## Claims

1. Linear actuator (10) for selectively actuating an actuatable device, the linear actuator (10) comprising:
a support;
a piston mounted on the support for movement generally along an actuation axis (AA) through a range of motion including an actuating position and a non-actuating position, the piston being movable in a first axial direction extending from the actuating position toward the non-actuating position and a second axial direction opposite the first axial direction, the piston including at least one retainer element and being configured to be mechanically connected to a movable input member of the actuatable device to position the input member at an actuated position;
an automatic driver (16) configured to selectively drive movement of the piston to the actuating position and the non-actuating position using power supplied by a power source;
the linear actuator (10) being **characterised in that** it further comprises
a manual driver (18) configured to selectively drive movement of the piston to the actuating position and the non-actuating position using a manual force, the manual driver (18) comprising:
a manual input member rotatably mounted on the support for selective rotation about the axis with respect to the support; and
a linking member threadably engaged with the manual input member for movement relative to the manual input member along the actuation axis (AA) when the manual input member is rotated relative to the linking member about the axis, the linking member including at least one retainer formation (84) configured for interlocking engagement with the at least one retainer element of the output assembly,
the interlocking engagement between the retainer formation (84) and the retainer element being operative to:
inhibit relative rotation between the piston and the linking member about the actuation axis (AA);
inhibit movement of the piston relative to the linking member along the axis in one of the first and second axial directions; and
permit movement of the piston relative to the linking member along the axis in the other of the first and second axial directions.

2. Linear actuator (10) as set forth in claim 1 wherein the linking member comprises an annular body having a radially inward facing surface and a radially outward facing surface, one of the radially inward and radially outward facing surfaces of the annular body defining a thread for threadably engaging the manual input member and the other of the radially inward and radially outward facing surface of the annular body defining the at least one retainer formation (84).

3. Linear actuator (10) as set forth in either of claims 1 and 2 wherein the at least one retainer formation (84) has at least one axial end surface oriented transverse to the actuation axis (AA) and at least one circumferential side surface extending generally along the axis.

4. Linear actuator (10) as set forth in claim 3 wherein the at least one retainer element defines at least one axial end surface oriented transverse to the actuation axis (AA) for opposingly engaging the at least one axial end surface of the at least one retainer formation (84) and defines at least one circumferential side surface extending generally along the axis for slidably engaging the at least one circumferential side surface (86) of the retainer formation (84) as the piston moves relative the linking member along the axis.

5. Linear actuator (10) as set forth in claim 4 wherein the at least one circumferential side surface of the at least one retainer element (84) includes at least one pair of opposing circumferential side surfaces (86) and the at least one retainer formation (84) includes at least one pair of oppositely facing circumferential side surfaces slidably received between the at least one pair of opposing circumferential side surfaces.

6. Linear actuator (10) as set forth in claim 5 wherein the piston further includes a radially facing surface between the at least one pair of opposing circumferential side surfaces (86) and extending from the at least one axial end surface of the at least one retainer element generally along the actuation axis (AA) at a substantially constant radial position.

7. Linear actuator (10) as set forth in any of claims 4-6 further comprising a spring (49) operatively received between the piston and the support to impart a biasing force on the piston that biases the at least one axial end surface of the at least one retainer element toward engagement with the at least one axial end surface (88) of the at least one retainer formation (84).

8. Linear actuator (10) as set forth in any of claims 1-5 wherein the piston is operatively engaged with the support to inhibit relative rotation between the piston and the support about the actuation axis (AA).

9. Linear actuator (10) as set forth in any of claims 1-6 wherein the piston includes an indicator member (40) positioned so that it is visible from a vantage external to the support when the piston is in one of the actuating position and the non-actuating position and positioned so that it is concealed by the support from said vantage when the output member (42) is in the other of the actuating position and the non-actuating position

## Patentansprüche

1. Linearer Aktuator (10) zum selektiven Betätigen einer betätigbaren Vorrichtung, wobei der lineare Aktuator (10) umfasst:
einen Träger;
einen Kolben, der an dem Träger für die Bewegung generell entlang einer Betätigungsachse (AA) durch einen Bewegungsbereich mit einer Betätigungsposition und einer Nicht-Betätigungsposition montiert ist, wobei der Kolben in einer ersten axialen Richtung, die sich von der Betätigungsposition in Richtung der Nicht-Betätigungsposition erstreckt, und einer zweiten axialen Richtung entgegengesetzt zur ersten axialen Richtung beweglich ist, wobei der Kolben mindestens ein Halterungselement aufweist und konfiguriert ist, um mechanisch mit einem beweglichen Eingangselement der betätigbaren Vorrichtung verbunden zu sein, um das Eingangselement in einer betätigten Position zu positionieren;
einen automatischen Treiber (16), der konfiguriert ist, um die Bewegung des Kolbens selektiv in die Betätigungsposition und die Nicht-Betätigungsposition mittels Energie zu treiben, die von einer Energiequelle geliefert wird;
wobei der lineare Aktuator (10) **dadurch gekennzeichnet ist, dass** er weiter umfasst:
einen manuellen Treiber (18), der konfiguriert ist, um die Bewegung des Kolbens selektiv in die Betätigungsposition und die Nicht-Betätigungsposition unter Verwendung einer manuellen Kraft zu treiben, wobei der manuelle Treiber (18) umfasst:
ein manuelles Eingabeelement, das drehbar auf dem Träger gelagert ist, um eine selektive Drehung um die Achse in Bezug auf den Träger zu ermöglichen; und
ein Verbindungselement, das gewindemäßig mit dem manuellen Eingabeelement in Eingriff steht, um sich in Bezug auf das manuelle Eingabeelement entlang der Betätigungsachse (AA) zu bewegen, wenn das manuelle Eingabeelement in Bezug auf das Verbindungselement um die Achse gedreht wird, wobei das Verbindungselement mindestens eine Halterungsanordnung (84) umfasst, die für einen Verriegelungseingriff mit dem mindestens einen Halterungselement der Ausgangsanordnung konfiguriert ist,
wobei der Verriegelungseingriff zwischen der Halterungsanordnung (84) und dem Halterungselement funktionsfähig ist:
um eine relative Drehung zwischen dem Kolben und dem Verbindungselement um die Betätigungsachse (AA) zu verhindern;
um die Bewegung des Kolbens in Bezug auf das Verbindungselement entlang der Achse hinsichtlich der ersten und zweiten axialen Richtung in die eine Richtung zu verhindern; und
um eine Bewegung des Kolbens in Bezug auf das Verbindungselement entlang der Achse hinsichtlich der ersten und zweiten axialen Richtung in die andere Richtung zu erlauben.

2. Linearer Aktuator (10) nach Anspruch 1, wobei das Verbindungselement einen Ringkörper mit einer radial nach innen gerichteten Oberfläche und einer radial nach außen gerichteten Oberfläche umfasst, wobei von der radial nach innen und radial nach außen gerichteten Oberfläche des Ringkörpers die eine ein Gewinde zum gewindefähigen Eingriff mit dem manuellen Eingabeelement definiert und von der radial nach innen und radial nach außen gerichteten Oberfläche des Ringkörpers die andere die mindestens eine Halterungsanordnung (84) definiert.

3. Linearer Aktuator (10) nach einem der Ansprüche 1 und 2, wobei die mindestens eine Halterungsanordnung (84) mindestens eine axiale Endfläche, die quer zur Betätigungsachse (AA) ausgerichtet ist, und mindestens eine Umfangsseitenfläche aufweist, die sich generell entlang der Achse erstreckt.

4. Linearer Aktuator (10) nach Anspruch 3, wobei das mindestens eine Halterungselement mindestens eine axiale Endfläche definiert, die quer zur Betätigungsachse (AA) ausgerichtet ist, um die mindestens eine axiale Endfläche der mindestens einen Halterungsanordnung (84) entgegengesetzt in Eingriff zu nehmen, und mindestens eine Umfangsseitenfläche definiert, die sich generell entlang der Achse erstreckt, um gleitend in die mindestens eine Umfangsseitenfläche (86) der Halterungsanordnung (84) einzugreifen, wenn sich der Kolben relativ zum Verbindungselement entlang der Achse bewegt.

5. Linearer Aktuator (10) nach Anspruch 4, wobei die mindestens eine Umfangsseitenfläche des mindestens einen Halterungselements (84) mindestens ein Paar gegenüberliegende Umfangsseitenflächen (86) aufweist und die mindestens eine Halterungsanordnung (84) mindestens ein Paar gegenüberliegender Umfangsseitenflächen aufweist, die gleitend zwischen dem mindestens einen Paar gegenüberliegender Umfangsseitenflächen aufgenommen sind.

6. Linearer Aktuator (10) nach Anspruch 5, wobei der Kolben weiter eine radial gerichtete Fläche zwischen dem mindestens einen Paar gegenüberliegender Umfangsseitenflächen (86) aufweist und sich von der mindestens einen axialen Endfläche des mindestens einen Halterungselements generell entlang der Betätigungsachse (AA) in einer im Wesentlichen konstanten radialen Position erstreckt.

7. Linearer Aktuator (10) nach einem der Ansprüche 4-6, weiter umfassend eine Feder (49), die funktionsfähig zwischen dem Kolben und dem Träger aufgenommen ist, um dem Kolben eine Vorspannkraft zu verleihen, die die mindestens eine axiale Endfläche des mindestens einen Halterungselements in Richtung auf den Eingriff mit der mindestens einen axialen Endfläche (88) der mindestens einen Halterungsanordnung (84) vorspannt.

8. Linearer Aktuator (10) nach einem der Ansprüche 1-5, wobei der Kolben funktionsfähig mit dem Träger in Eingriff steht, um eine relative Drehung zwischen dem Kolben und dem Träger um die Betätigungsachse (AA) zu verhindern.

9. Linearer Aktuator (10) nach einem der Ansprüche 1-6, wobei der Kolben ein Anzeigeelement (40) aufweist, das so positioniert ist, dass es von einem Blickwinkel außerhalb des Trägers sichtbar ist, wenn sich der Kolben hinsichtlich der Betätigungsposition und der Nicht-Betätigungsposition in der einen befindet, und so positioniert ist, dass es durch den Träger vor dem Blick verborgen ist, wenn sich das Ausgangselement (42) hinsichtlich der Betätigungsposition und der Nicht-Betätigungsposition in der anderen befindet.

## Revendications

1. Actionneur linéaire (10) pour actionner sélectivement un dispositif actionnable, l'actionneur linéaire (10) comprenant :
un support ;
un piston monté sur le support pour un mouvement généralement le long d'un axe d'actionnement (AA) à travers une plage de déplacement incluant une position d'actionnement et une position de non-actionnement, le piston étant déplaçable dans une première direction axiale s'étendant depuis la position d'actionnement vers la position de non-actionnement et dans une deuxième direction axiale opposée à la première direction axiale, le piston incluant au moins un élément de retenue et étant configuré pour être mécaniquement relié à un élément d'entrée déplaçable du dispositif actionnable pour positionner l'élément d'entrée à une position actionnée ;
un pilote automatique (16) configuré pour piloter sélectivement un mouvement du piston jusqu'à la position d'actionnement et la position de non-actionnement en utilisant une puissance alimentée par une source de puissance ;
l'actionneur linéaire (10) étant **caractérisé en ce qu'**il comprend en outre un pilote manuel (18) configuré pour entraîner sélectivement un mouvement du piston jusqu'à la position d'actionnement et la position de non-actionnement en utilisant une force manuelle, le pilote manuel (18) comprenant :
un élément d'entrée manuel monté en rotation sur le support pour une rotation sélective autour de l'axe par rapport au support ; et
un élément de liaison engagé par vissage avec l'élément d'entrée manuel pour un mouvement relativement à l'élément d'entrée manuel le long de l'axe d'actionnement (AA) quand l'élément d'entrée manuel est mis en rotation relativement à l'élément de liaison autour de l'axe, l'élément de liaison incluant au moins une formation de retenue (84) configurée pour un engagement interverrouillé avec ledit au moins un élément de retenue de l'assemblage de sortie,
l'engagement interverrouillé entre la formation de retenue (84) et l'élément de retenue étant opérationnel pour :
inhiber une rotation relative entre le piston et l'élément de liaison autour de l'axe d'actionnement (AA) ;
inhiber un mouvement du piston relativement à l'élément de liaison le long de l'axe dans l'une de la première et de la deuxième direction axiale ; et
permettre un mouvement du piston relativement à l'élément de liaison le long de l'axe dans l'autre de la première et de la deuxième direction axiale.

2. Actionneur linéaire (10) selon la revendication 1, dans lequel l'élément de liaison comprend un corps annulaire ayant une surface faisant face radialement vers l'intérieur et une surface faisant face radialement vers l'extérieur, une des surfaces faisant face radialement vers l'intérieur et radialement vers l'extérieur du corps annulaire définissant un pas de vis pour engager par vissage l'élément d'entrée manuel, et l'autre de la surface faisant face radialement vers l'intérieur et radialement vers l'extérieur du corps annulaire définissant ladite au moins une formation de retenue (84).

3. Actionneur linéaire (10) selon la revendication 1 ou 2, dans lequel ladite au moins une formation de retenue (84) a au moins une surface terminale axiale orientée transversalement à l'axe d'actionnement (AA) et au moins une surface latérale circonférentielle s'étendant généralement le long de l'axe.

4. Actionneur linéaire (10) selon la revendication 3, dans lequel ledit au moins un élément de retenue définit au moins une surface terminale axiale orientée transversalement à l'axe d'actionnement (AA) pour engager à l'opposé ladite au moins une surface terminale axiale de ladite au moins une formation de retenue (84), et définit au moins une surface latérale circonférentielle s'étendant généralement le long de l'axe pour engager par coulissement ladite au moins une surface latérale circonférentielle (86) de la formation de retenue (84) quand le piston se déplace relativement à l'élément de liaison le long de l'axe.

5. Actionneur linéaire (10) selon la revendication 4, dans lequel ladite au moins une surface latérale circonférentielle dudit au moins un élément de retenue (84) inclut au moins une paire de surfaces latérales circonférentielles opposées (86), et ladite au moins une formation de retenue (84) inclut au moins une paire de surfaces latérales circonférentielles se faisant face à l'opposé reçues par coulissement entre ladite au moins une paire de surfaces latérales circonférentielles opposées.

6. Actionneur linéaire (10) selon la revendication 5, dans lequel le piston comprend en outre une surface faisant face radialement entre ladite au moins une paire de surfaces latérales circonférentielles opposées (86) et s'étendant depuis ladite au moins une surface terminale axiale dudit au moins un élément de retenue généralement le long de l'axe d'actionnement (AA) à une position radiale sensiblement constante.

7. Actionneur linéaire (10) selon l'une quelconque des revendications 4 à 6, comprenant en outre un ressort (49) reçu de manière opérationnelle entre le piston et le support pour imposer une force de sollicitation sur le piston qui sollicite ladite au moins une surface terminale axiale dudit au moins un élément de retenue vers un engagement avec ladite au moins une surface terminale axiale (88) de ladite au moins une formation de retenue (84).

8. Actionneur linéaire (10) selon l'une quelconque des revendications 1 à 5, dans lequel le piston est engagé de manière opérationnelle avec le support pour inhiber une rotation relative entre le piston et le support autour de l'axe d'actionnement (AA).

9. Actionneur linéaire (10) selon l'une quelconque des revendications 1 à 6, dans lequel le piston inclut un élément d'indication (40) positionné de manière à être visible depuis un repère extérieur au support quand le piston est dans l'une des positions d'actionnement et de non-actionnement et est positionné de manière à être caché par le support depuis ledit repère quand l'élément de sortie (42) est dans l'autre de la position d'actionnement et de non-actionnement.
